# EUROPÄISCHE PATENTANMELDUNG

(11) **EP 0 702 020 A1**
(43) Veröffentlichungstag der Anmeldung: **20.03.1996**
(21) Anmeldenummer: 95110916.4
(22) Anmeldetag: 12.07.1995
(51) Int. Cl.: C07H 15/04

(54) **Verfahren zur Bleichung von Alkylpolyglycosiden**

(30) Priorität: 07.09.1994 DE 4431852
(71) Anmelder: HÜLS AKTIENGESELLSCHAFT, D-45764 Marl (DE)
(72) Erfinder: Grützke, Jürgen, D-44803 Bochum (DE); Schmidt, Stefan, Dr., D-45721 Haltern (DE)

(57) **Zusammenfassung**

Die Anmeldung betrifft ein neues Verfahren zur Bleichung von Alkylpolyglycosiden mit C₈- bis C₂₀-Alkylgruppen in wäßriger Lösung mit Persauerstoffverbindungen. Die Bleichung wird dabei in einem Rohrreaktor durchgeführt.

## Beschreibung

Die Erfindung betrifft ein Verfahren zur Bleichung von Alkylpolyglycosiden, bei denen die Alkylgruppen 8 bis 24 C-Atome aufweisen, in wäßriger Lösung durch Persauerstoffverbindungen.

Alkylpolyglycoside sind ungiftige und leicht abbaubare oberflächenaktive Stoffe. Sie werden deshalb als Wasch- und Reinigungsmittel und als Emulgatoren und Dispergatoren verwendet. Sie haben aber nur dann die gewünschten Grenzflächeneigenschaften, wenn die Alkylgruppen mindestens 8 C-Atome aufweisen.

Alkylpolyglycoside mit langkettigen Alkylgruppen werden im allgemeinen durch ein- oder mehrstufige Synthesen hergestellt.

Ein einstufiges Herstellverfahren wird u. a. in DE-A-41 01 252 beschrieben.

Ein zweistufiges Verfahren wird beispielsweise in EP-A-0 306 652 angegeben, wonach man zunächst durch Glycosidierung mit n-Butanol ein n-Butylglycosid und darauf durch Umglycosidierung mit einem langkettigen Alkohol das gewünschte langkettige Alkylpolyglycosid herstellt.

Während der Herstellung der Alkylpolyglycoside kommt es als Folge von unerwünschten Oxidations- und Kondensationsprozessen zu Produktverfärbungen. Die anwendungstechnischen Eigenschaften werden dadurch nicht berührt. Der nachteilige optische Eindruck erschwert jedoch die Vermarktung der Produkte. Um die Farbqualität zu verbessern, werden deshalb Alkylpolyglycoside nach ihrer Herstellung im allgemeinen einem Bleichprozeß unterworfen. Dazu wird zunächst neutralisiert und überschüssiger Fettalkohol abdestilliert, worauf dann die Bleichung in einer wäßrigen Lösung vorgenommen wird.

Nach EP-A-0 165 721 kann die Farbe von Alkylpolyglycosid-Lösungen durch mehrstufige Bleichung mit Wasserstoffperoxid verbessert und durch Zusatz von Schwefeldioxid freisetzenden Verbindungen stabilisiert werden. Wegen der hohen Flüchtigkeit des Wasserstoffperoxids wird die Bleichung dabei in einem geschlossenen Behälter bei 1 bis 20 bar durchgeführt.

Üblicherweise erfolgt die Bleichung in Rührkesseln. Diese Reaktoren haben aber für eine kontinuierliche Fahrweise ein ungünstig breites Verweilzeitspektrum und führen zu einem breiten Spektrum an Reaktionsprodukten. Dadurch wird hier zur Vermeidung der Nachteile ein hoher zusätzlicher apparativer Aufwand erforderlich.

Aufgabe der vorliegenden Erfindung war es, ein verbessertes Verfahren zur Bleichung bereitzustellen. Im besonderen sollten dabei die Nachteile der Bleichung in den üblichen Apparaturen des Standes der Technik vermieden werden.

Erfindungsgemäß wird die Aufgabe dadurch gelöst, daß man die Bleichung in einem Rohrreaktor durchführt.

Geeignete Rohrreaktoren haben im allgemeinen einen Durchmesser von 0,5 bis 50 cm und eine Länge von 0,5 bis 50 m, wobei die Länge mindestens das 4fache des Durchmessers sein soll. Im einfachsten Falle handelt es sich dabei um ein beheizbares Rohr. Vorzugsweise weist der Rohrreaktor jedoch auch mehrere, meist 1 bis 20, Einleitungsstellen auf. Über diese Einleitungsstellen können dann Bleichmittel und Lauge zur pH-Einstellung nachdosiert werden.

Der Rohrreaktor kann auch Schikanen und Verwirbelungseinbauten, beispielsweise Inline-Mischer, enthalten. Das Rohr kann horizontal, schräg oder vertikal angeordnet sein. So werden Substrat und Bleichmittel trotz Gleichstrombedingungen sehr gut miteinander vermischt. Dem Rohrreaktor können auch Rührkessel vor- und nachgeschaltet sein.

Geeignete Bleichmittel sind beispielsweise Wasserstoffperoxid, Ozon, Perborat, Natriumhypochlorit, Percarbonat, Peracetat oder Peroxodisulfat. Dabei wird Wasserstoffperoxid vorzugsweise verwendet.

Die wäßrigen Alkylpolyglycosid-Lösungen sind vorzugsweise 30- bis 90%ig. Dabei werden 40- bis 70%ige Lösungen besonders bevorzugt für die Bleichung eingesetzt.

Bei der Herstellung der Alkylpolyglycoside können als Saccharide beispielsweise Glucose, Mannose, Galaktose, Talose oder Fructose eingesetzt worden sein. Das bevorzugte Saccharid ist dabei Glucose.

Die Alkylketten leiten sich von Alkoholen wie beispielsweise Octanol, Decanol, Laurylalkohol, Myristyl-, Palmityl- und Stearylalkohol her.

Dabei weisen die Alkylpolyglycoside im allgemeinen einen mittleren Glycosidierungsgrad von 1 bis 5 auf, wobei Produkte mit mittleren Glycosidierungsgraden von 1,1 bis 2 vorzugsweise eingesetzt werden.

Die Bleichung erfolgt meist bei 50 bis 120 °C und einem Druck von 1 bis 10 bar.

Nach dem vorliegenden Verfahren können Alkylpolyglycoside bei einer engen Verweilzeitverteilung einheitlich gebleicht werden. Bleichmittel und weitere Hilfsstoffe können dabei in einer einfachen Apparatur genau dosiert werden. Das Bleichverfahren kann diskontinuierlich und problemlos auch kontinuierlich durchgeführt werden. Dabei erhält man (bei 50%igen Lösungen) hellfarbige Produkte mit Iodfarbzahlen von unter 20.

Das folgende Beispiel soll die Erfindung verdeutlichen.

### Beispiel 1

Einem beheizbaren Rohr (Durchmesser: 50 mm, Länge: 1,5 m) werden stündlich 20 kg einer wäßrigen Alkylpolyglycosid-Lösung (Wasseranteil: 43 %, Iodfarbzahl: 60, pH-Wert: 8) und 0,6 kg einer 35%igen H₂O₂-Lösung zugeführt. Dem Rohrreaktor ist ein Static-Mischer vorgeschaltet, in dem beide Lösungen homogen vermischt werden. Die mittlere Reaktorinnentemperatur wird durch eine Rohrbeheizung auf 75 °C eingestellt. Der pH-Wert wird durch Zugabe von Natronlauge durch 2 Stutzen nach einem und nach zwei Drittel der Reaktorlänge annähernd konstant gehalten. 2 Inline-Mischer sorgen für eine ausreichende Homogenisierung im Reaktor.

Am Austritt des Rohrreaktors besitzt die wäßrige Alkylpolyglycosid-Lösung eine Iodfarbzahl von 5.

## Patentansprüche

1. Verfahren zur Bleichung von Alkylpolyglycosiden mit C₈- bis C₂₀-Alkylgruppen in wäßriger Lösung mit Persauerstoffverbindungen,
dadurch gekennzeichnet,
daß man die Bleichung in einem Rohrreaktor durchführt.

2. Verfahren nach Anspruch 1,
dadurch gekennzeichnet,
daß man die Bleichung in einem Rohrreaktor mit 1 bis 20 Einleitungsstellen vornimmt und dabei Bleichmittel und Lauge über diese Einleitungsstellen nachdosiert.

3. Verfahren nach Anspruch 1,
dadurch gekennzeichnet,
daß man in einem Rohrreaktor mit Inline-Mischern bleicht.

4. Verfahren nach Anspruch 1,
dadurch gekennzeichnet,
daß man mit Wasserstoffperoxid bleicht.

5. Verfahren nach Anspruch 1,
dadurch gekennzeichnet,
daß man für die Bleichung eine 30- bis 90%ige, vorzugsweise eine 40- bis 70%ige, wäßrige Alkylpolyglycosid-Lösung einsetzt.
